# EUROPEAN PATENT APPLICATION

(11) **EP 3 978 509 A1**
(43) Date of publication of application: **06.04.2022**
(21) Application number: 19937307.7
(22) Date of filing: 15.08.2019
(51) Int. Cl.: C07K 7/06, A61K 38/08, A61P 19/08, A61P 19/10

(54) **BONE REMODELING REGULATORY POLYPEPTIDES AND USE**

(30) Priority: 10.07.2019 CN 201910621375
(71) Applicant: The Second Xiangya Hospital of Central South University, Changsha Hunan 410011 (CN)
(72) Inventor: ZHOU, Houde, Changsha, Hunan 410011 (CN); GUO, Yue, Changsha, Hunan 410011 (CN); ZHOU, Yinghui, Changsha, Hunan 410011 (CN); YUE, Haiqng, Changsha, Hunan 410011 (CN)
(74) Representative: Herrmann, Uwe
(86) International application number: PCT/CN2019/100736
(87) International publication number: WO 2021/003800

(57) **Abstract**

This invention provided a series of bone remodeling regulatory peptides and application thereof. On the basis of our initial experiments, through sequence alignment, structure analysis, physical and chemical properties and function prediction, we designed a series of bone remodeling regulatory peptides and synthesized them by solid-phase peptide synthesis method. These peptides have the features of short length, simple synthesis, low cost, low cytotoxicity, high biological stability, moderate half-life, good bone targeting. They can regulate the differentiation and function of osteoclasts and osteoblasts by adjusting the concentration, thereby achieving the orderly regulation of bone resorption and bone formation, and have wide potential application prospects.

## Description

### TECHNICAL FIELD

The present invention belongs to the technical field of bone remodeling regulation and particularly relates to bone remodeling regulatory peptides and application thereof.

### BACKGROUND

The development and therapeutic targets of the existing anti-osteoporosis drugs mainly focus on bone remodeling (the dynamic balance between bone formation and bone resorption), that is, how to inhibit bone resorption or promote bone formation. Based on these two points, the anti-osteoporosis drugs in clinic may be divided into anti-bone resorption drugs, such as diphosphonate and calcitonin, and bone formation-promoting drugs, such as teriparatide. However, the former inhibits bone resorption as well as bone formation at the same time, which significantly increases the incidence of adverse reactions caused by long-term use; and the latter promotes bone formation as well as bone resorption at the same time, which may increase the incidence of fracture after 2 years of use. Up to now, there are no anti-bone resorption drugs that only inhibit bone resorption without inhibiting bone formation, and there are also no bone formation-promoting drugs that only promote bone formation without promoting bone resorption! These greatly limited the effectiveness and application scope of clinical drugs. At present, bone remodeling regulatory drugs mainly include bisphosphonates, calcitonin, selective estrogen receptor modulators, parathyroid hormone analogs, RANKL inhibitors, and the like. Only the parathyroid hormone analogs are bone remodeling regulatory peptide drugs, which are derived from parathyroid hormone, which has bone remodeling regulating effect itself. At present, there are no non-hormone based peptide drugs at home and abroad.

### SUMMARY

Bone tissues are composed of bone matrix and cells, and 90% of the organic component of the bone matrix is type I collagen. Although collagen has been widely used in bone tissue engineering materials, cell culture, medical beauty and even daily life, it has also been questioned. The reasons mainly focus on the following two aspects: first, because type I collagen is a collagen fiber bundle with a triple helical structure and has a large molecular weight, it has long been considered to only play a role in supporting, protecting, or anchoring cytokine through a structural gap. Second, the structure of type I collagen is complex, it's a heterotrimeric structural protein composed of two α1 chains and one α2 chain. Thus, the core sequence of type I collagen binding to its receptor is difficult to find, and its active effect cannot be explained from molecular mechanism, which leads to doubts about the application of collagen products in health care and medical treatment, and few drugs developed with collagen as targets are available. Therefore, identifying and designing core type I collagen peptides binding to the receptors and revealing a mechanism of the type I collagen in regulating bone remodeling will not only establish a new mechanism to further refine regulation of bone metabolism, but will also provide new evidence for application of collagen and its peptides.

In addition, in the treatment of metabolic bone diseases such as osteoporosis, achieving specific bone targeting of drugs can significantly increase the therapeutic effect. Although bisphosphonates can specifically target bone formation and bone resorption surfaces by binding to hydroxyapatite (HA) on bone surfaces, long-term or massive application of bisphosphonates may lead to occurrence of osteonecrosis. However, oligopeptides such as glutamic acid (Glu) and aspartic acid (ASP) not only have the characteristics of being enzymatically degradable, not forming colloids with metals and having no long-term damage to health, but also can sensitively sense changes of crystallinity in HA on the bone resorption surfaces, so as to achieve targeting of the bone resorption surfaces. However, uncertainty of the effect of bone targeting often increases due to the number of connected oligopeptides and differences in the structures of the connected peptides.

Through sequence alignment, structural analysis and validation experiments of physical and chemical properties and functions, the inventors have newly discovered bone remodeling regulatory peptides, and synthesized these peptides by solid-phase peptide synthesis method. These peptides are composed of 9 amino acids and are easy to synthesize with low cost. The bone remodeling regulatory peptides of the present invention have the features of low cytotoxicity, high biological stability, and moderate half-life and have a wide potential application prospect. It has been experimentally confirmed that the bone remodeling regulatory peptides can concentration-specifically regulate the differentiation and function of osteoclasts and osteoblasts to achieve orderly regulation of bone resorption and bone formation. On this basis, we designed and synthesized bone remodeling regulatory peptides that can target bone tissues using aspartic acid (Asp) 8 and validated their bone-targeting ability *in vivo,* which are expected to develop new clinical drugs against metabolic bone diseases such as osteoporosis and osteosclerosis.

The objective of the present invention is to provide new bone remodeling regulatory peptides and application thereof. These peptides have the features of short length, simple synthesis, low cost, low cytotoxicity, high biological stability, moderate half-life, and good bone targeting, and have a wide potential application prospect. The bone remodeling regulatory peptides of the present invention can concentration-specifically regulate the differentiation and function of osteoclasts and osteoblasts to achieve orderly regulation of bone resorption and bone formation.

The core sequence of the bone remodeling regulatory peptides in the present invention is any one or more of the following:
Gly-Xaa-Pro-Gly-Xaa-Xaa-Gly-Xaa-Xaa, as shown in SEQ ID No. 1.

The second amino acid Xaa is Ala or Pro or Gly, the fifth amino acid Xaa is Pro or Ala, the sixth amino acid Xaa is Ala or Gln or Thr or Ser or hydroxylated Pro, the eighth amino acid Xaa is Phe or Ser or Asp or Tyr, the ninth amino acid Xaa is Ala or Gln or Pro or Arg or hydroxylated Pro, and the third amino acid Pro is hydroxylated. The hydroxylated Pro is hydroxyproline Hyp.

Further, the present invention prefers one or more of the above core sequences, specifically as follows:
A1-1: Gly-Ala-Pro-Gly-Pro-Gln-Gly-Phe-Gln, as shown in SEQ ID No. 2.
A1-2-1: Gly-Ala-Pro-Gly-Ala-Pro-Gly-Ser-Gln, as shown in SEQ ID No. 3.
A1-4-1: Gly-Pro-Pro-Gly-Pro-Ala-Gly-Phe-Ala, as shown in SEQ ID No. 4.
A1-5-3: Gly-Pro-Pro-Gly-Ala-Thr-Gly-Phe-Pro, as shown in SEQ ID No. 5.
OSC^{pep}: Gly-Ala-Pro-Gly-Pro-Ala-Gly-Phe-Ala, as shown in SEQ ID No. 6.

Furthermore, the present invention prefers one or more of the above core sequences, specifically as follows:
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala,
A1-5-3: Gly-Pro-Pro-Gly-Ala-Thr-Gly-Phe-Pro.

The bone remodeling regulatory peptides include a series of peptides having a bone remodeling regulating function obtained by N-terminal or C-terminal modification, extension of an amino acid sequence, substitution and replacement of amino acids, and cyclization or chiral transformation on the basis of the core sequence.

The bone remodeling regulating function refers to controlling the differentiation and function of osteoclasts and osteoblasts by regulating the concentration of bone remodeling regulatory peptides, thereby achieving the orderly regulation of bone resorption and bone formation.

According to the bone remodeling regulatory peptides, an amino end of the amino acid sequence is connected with at least 2 aspartic acids, preferably 8 consecutive aspartic acids.

Use of the bone remodeling regulatory peptides in preparing a preparation for promoting bone resorption and inhibiting bone formation, or a preparation for inhibiting bone resorption and promoting bone formation.

When the bone remodeling regulatory peptides are any one or more of the following:
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala, and
A1-5-3: Gly-Pro-Pro-Gly-Ala-Thr-Gly-Phe-Pro,

The concentration of the bone remodeling regulatory peptide preparation for promoting bone resorption and inhibiting bone formation is < 500 µg/mL, and preferably < 200 µg/mL; and the concentration of the bone remodeling regulatory peptide preparation for inhibiting bone resorption and promoting bone formation is ≥ 500 µg/mL, and preferably 500-1000 µg/mL.

When the bone remodeling regulatory peptides are OSC^{pep}: Gly-Ala-Pro-Gly-Pro-Ala-Gly-Phe-Ala, the concentration of the bone remodeling regulatory peptide preparation for promoting bone resorption and inhibiting bone formation is < 8000 µg/mL, and preferably 1000-5000 µg/mL; and the concentration of the bone remodeling regulatory peptide preparation for inhibiting bone resorption and promoting bone formation is ≥ 8000 µg/mL, and preferably 8000-10000 µg/mL.

The application of the bone remodeling regulatory peptide preparation including: the treatment of metabolic bone diseases comprising osteoporosis and osteosclerosis.

When the bone remodeling regulatory peptides are any one or more of
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala, and
A1-5-3: Gly-Pro-Pro-Gly-Ala-Thr-Gly-Phe-Pro,

The concentration of the bone remodeling regulatory peptide preparation for osteoporosis treatment is ≥ 500 µg/mL, and preferably 500-1000 µg/mL; and the concentration of the bone remodeling regulatory peptide preparation for osteosclerosis treatment is < 500 µg/mL, and preferably < 200 µg/mL.

Further, when the bone remodeling regulatory peptides are OSC^{pep}: Gly-Ala-Pro-Gly-Pro-Ala-Gly-Phe-Ala, the concentration of the bone remodeling regulatory peptide preparation for osteoporosis treatment is ≥ 8000 µg/mL, and preferably 8000-10000 µg/mL; and the concentration of the bone remodeling regulatory peptide preparation for osteosclerosis treatment is < 8000 µg/mL, and preferably 1000-5000 µg/mL.

Osteoclast associated receptor (OSCAR) is a collagen receptor that expressed on monocytes and osteoclasts, and has provided a great breakthrough in the mechanistic study of collagens. However, type I collagen belongs to a heterotrimer with complex peptide chain structure, and the mode of its interaction with OSCAR is unclear. The inventors studied protein structures of mouse OSCAR (mOSCAR) and found it has 5 highly conserved amino acid sites that can bind to collagen, so we speculated that type I collagen can combine with OSCAR to participate in bone remodeling. Since we found that osteoblasts and osteocytes, which participate in bone formation, also expressed high level of OSCAR through experiments, we further speculated that type I collagen could combine with OSCAR to participate in the regulation of not only bone resorption but also bone formation.

Therefore, through sequence alignment, structure analysis, and validation of physical and chemical properties and functions, we innovatively designed and synthesized 4 segments of peptides derived from the type I collagen and 1 segment of peptide derived from types II and III collagen, which include 9 amino acid core sequences using a solid-phase peptide synthesis method, and the 5 segments of peptides are collectively called the bone remodeling regulatory peptides. It is found that the newly synthesized 5 segments of bone remodeling regulatory peptides have low cytotoxicity, high biological stability, and appropriate half-life. Then, we used mouse primary bone marrow mononuclear cells (BMMs) to construct an *in vitro* osteoclast differentiation model under induction of RANKL and M-CSF, and used bovine bone slices and BMMs to construct a bone resorption model under induction of RANKL and M-CSF. WGA staining was used to observe the formation of bone resorption lacunae and showed that type I collagen bone remodeling regulatory peptides (A1-1/A1-2-1/A1-4-1/A1-5-3) can promote osteoclast differentiation and bone resorption at a low concentration (< 500 µg/mL, as low as 200 µg/mL or below has a good effect), and inhibit osteoclast differentiation and bone resorption at a high concentration (≥ 500 µg/mL, 500-1000 µg/mL may lead to a significant effect). The bone remodeling regulatory peptide derived from type II and III collagen (OSC^{pep}) has the same concentration-specific regulating effect on bone resorption, but its effective low concentration is < 8000 µg/mL (1000-5000 µg/mL leads to a significant effect), and its effective high concentration is ≥ 8000 µg/mL (preferably 8000-10000 µg/mL). These indicates that the type I collagen bone remodeling regulatory peptides have higher biological activity. Primary pre-osteoblasts derived from the skulls of newborn mice were induced by β-sodium glycerophosphate and vitamin C to establish a bone formation model. Alkaline phosphatase (ALP) staining was used to evaluate the differentiation of osteoblasts and showed that a low concentration of type I collagen bone remodeling regulatory peptides (< 500 µg/mL, preferably 200 µg/mL) can inhibit osteoblast differentiation, while a high concentration of type I collagen bone remodeling regulatory peptides (≥ 500 µg/mL, preferably 500-1000 µg/mL) can promote osteoblast differentiation. When the bone remodeling regulatory peptides are OSC^{pep}, the concentration of < 8000 µg/mL (preferably 1000-5000 µg/mL) can inhibit osteoblast differentiation, while the concentration of ≥ 8000 µg/mL (preferably 8000-10000 µg/mL) can promote osteoblast differentiation. Therefore, it can be concluded that the bone remodeling regulatory peptides can concentration-specifically regulate bone remodeling.

After confirming the effect of the bone remodeling regulatory peptides on bone formation/bone resorption for the first time, we will further explore the feasibility of clinical application of the bone remodeling regulatory peptides. If the bone remodeling regulatory peptides can specifically target bone tissues, the therapeutic effect may be significantly increased. Among the existing osteoporosis drugs, the bisphosphonates approved by FDA can specifically target bone formation and bone resorption surfaces by binding to the hydroxyapatite. However, long-term or extensive application of the bisphosphonates may lead to osteonecrosis. While oligopeptides such as glutamic acid (Glu) and aspartic acid (Asp) have the advantages of being enzymatically degradable, not forming colloids with metals, and having no long-term damage to health. Different connecting numbers of Asps can lead to different bone targeting abilities. With the increase of Asps connection number, the binding ability of Asp to hydroxyapatite is enhanced, among which 8, 11, and 14 Asps have the best bone tissue targeting ability. In addition, Asp has two kinds of conformations: levorotary conformation (L-Asp) and dextrorotary conformation (D-Asp). It has been found that (D-Asp)n is more stable than (L-Asp)n. Therefore, the 5 segments of bone remodeling regulatory peptides of the present invention are connected by (D-Asp)8 and labeled by a red fluorophores Cy5 for it has stronger solubility, stability, and fluorescence intensity than conventional green fluorophores FITC. The *in vivo* experiment shows that compared with the normal saline and Cy5 control group, fluorescence is only found in femurs, tibias, vertebrae, skulls, and alveolar bones after 1-7 days of tail vein injection in the bone remodeling regulatory peptides labeled by Cy5 and connected by (D-Asp)8, indicated that the bone remodeling regulatory peptides have good bone tissue specificity. It is exciting that the bone-targeting bone remodeling regulatory peptides has a half-life of up to 7 days in bone, which is much higher than that of conventional (Asp)8 loaded drugs.

On the basis of our first experimental discovery in the early stage, 5 segments of bone remodeling regulatory peptides were designed and synthesized using the solid-phase peptide synthesis method after sequence alignment, structural analysis and validation of the physical and chemical properties and functions. These peptides are composed of 9 core amino acids and only one-fifth of the length of triple helix peptides derived from type II and III collagen; these peptides are easy to synthesize with low cost. The bone remodeling regulatory peptides of the present invention have the features of low cytotoxicity, high biological stability, moderate half-life, and strong ability to regulate bone resorption, and have a wide potential application prospect. It has been confirmed by *in vitro* experiments that the bone remodeling regulatory peptides can concentration-specifically regulate the differentiation and functions of osteoclasts and osteoblasts to achieve the orderly regulation of bone resorption and bone formation. On this basis, we designed and synthesized the bone-targeting bone remodeling regulatory peptides using the (Asp)8, and validated their specific bone tissue targeting ability *in vivo,* which are expected to develop new clinical drugs for the treatment of metabolic bone diseases such as osteoporosis.

### DETAILED DESCRIPTION

The following embodiments are used to further illustrate but not to limit the present invention.

### Embodiment 1

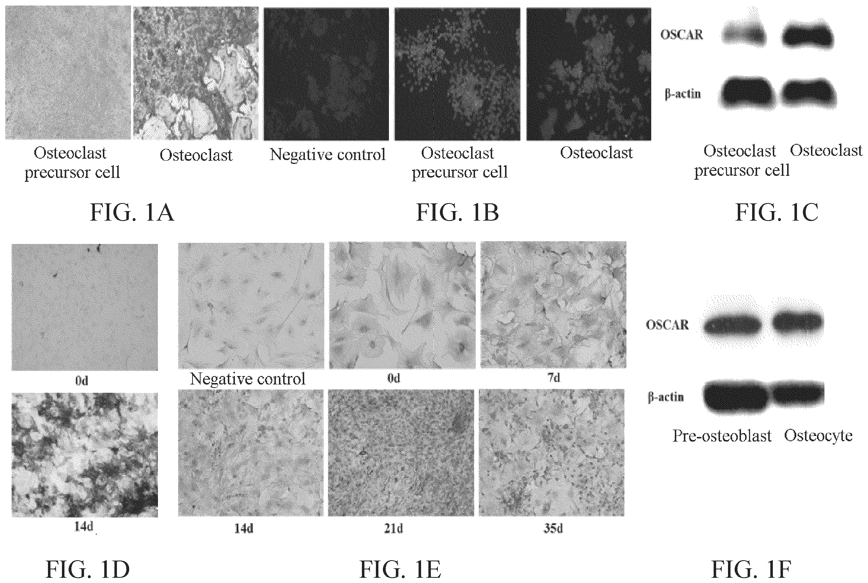

Expression of collagen receptor OSCAR on osteoclasts and osteoblasts was determined.
1) Bilateral femurs and tibias of wild-type female mice were aseptically isolated, and bone marrow cells were obtained by repeatedly washing bone marrow cavities with culture medium. After culturing in α-MEM complete medium containing M-CSF (30 ng/mL) for 24-hour, nonadherent cells were suctioned, centrifuged, and resuspended, and then cultured in the α-MEM complete medium containing M-CSF (30 ng/mL) for 3 days to obtain adherent mouse primary bone marrow mononuclear macrophages (osteoclast precursor cells). Osteoclast precursor cells were conducted by M-CSF (50 ng/mL) and RANKL (100 ng/mL) to obtain osteoclast and identified by TRAP staining (FIG. 1A). The expression of the collagen receptor OSCAR on osteoclast precursor cells and osteoclasts were determined by immunofluorescence (FIG. 1B) and Western blot (FIG. 1C).
2) After skulls of the wild-type mice were aseptically isolated, cells were isolated by collagenase digestion and nucleated cells were obtained by density gradient centrifugation. Cells were induced to differentiate into osteoblasts by medium containing 10 mM β-sodium glycerophosphate, 50 µM vitamin C and 10⁻⁷ M dexamethasone and identified by ALP staining (FIG. 1D). The expression of the collagen receptor OSCAR on pre-osteoblasts induced for 0th, 7th, 14th, 21st, and 35th day and MLO-Y4 bone cell lines was determined by immunohistochemistry (FIG. IE) and Western blot (FIG. IF).

### Embodiment 2

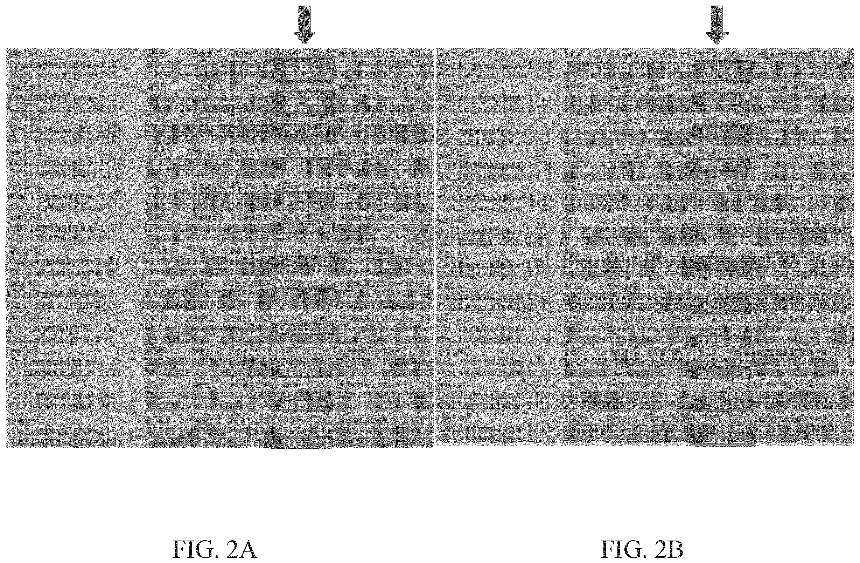

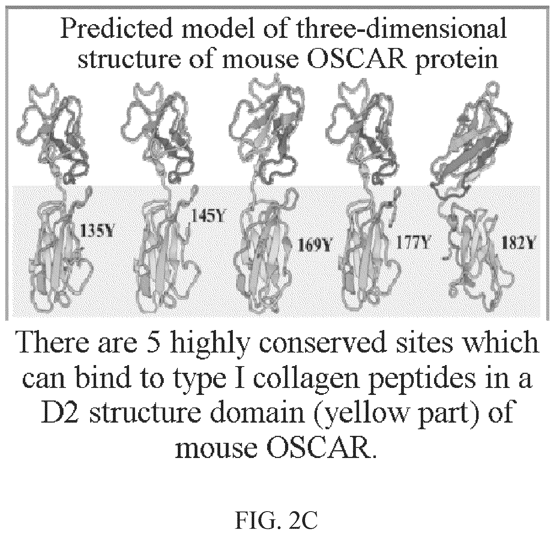

Multi-sequence alignment analysis was conducted on a type I collagen α1 subunit of humans (FIG. 2A) and type I collagen α2 subunit of mice (FIG. 2B) to find sequences that may bind to the collagen receptor OSCAR (box parts of FIG. 2A and B; in each group of sequences, the upper sequence represents Col1a1, and the lower sequence represents Col1a2). In addition, protein structure of mouse OSCAR (mOSCAR) were predicted, and there were 5 highly conserved amino acid sites in mOSCAR could bind to collagen (FIG. 2C).

### Embodiment 3

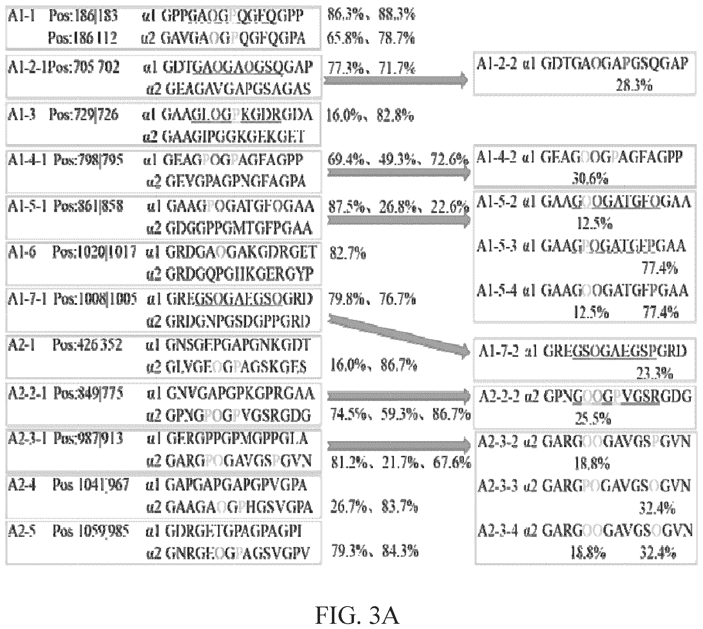

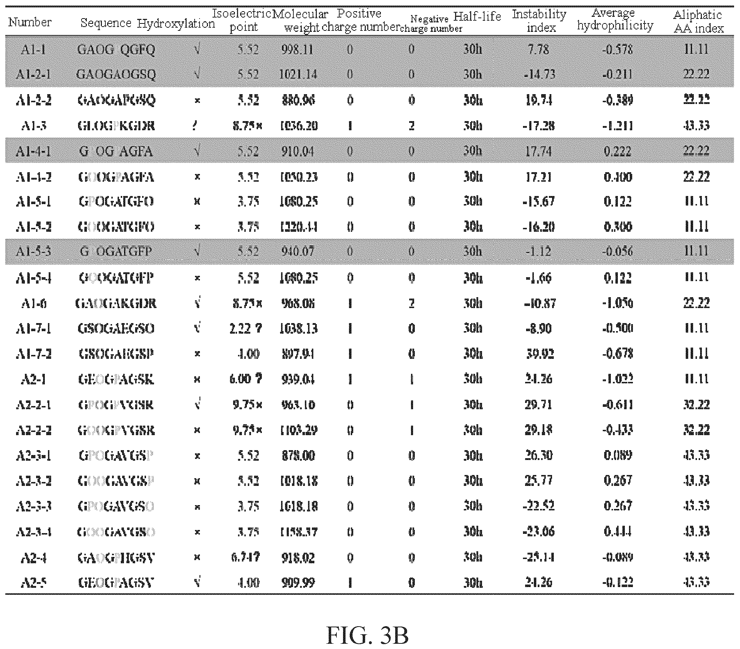

By predicting the physical and chemical properties and function (isoelectric points, molecular weights, positive and negative charge numbers, half-lives, instability indexes, average hydrophilicities, and aliphatic amino acid indexes), 4 segments of performance-stable sequences derived from type I collagen and 1 segment of sequence derived from type II and III collagen were screened and then synthesized using a solid-phase peptide synthesis method, that is 5 segments of bone remodeling regulatory peptides were synthesized.
1) By analyzing the amino acid hydroxylation level (FIG. 3A), electric points, positive and negative charge residues, atomic numbers, the half-lives, instability coefficients, average hydrophilicities, and aliphatic amino acid indexes (FIG. 3B) of the sequences that may bind to collagen receptor OSCAR in mouse type I collagen, 4 segments of performance-stable sequences (gray part in FIG. 3B) were screened through comprehensive analysis of the above parameters and named A1-1, A1-2-1, A1-4-1, and A1-5-3, respectively.
2) 4 segments of peptides derived from the type I collagen (A1-1, A1-2-1, A1-4-1, and A1-5-3) and 1 segment of the peptide (OSC^{pep}) derived from the type II and III collagen which can bind to the collagen receptor OSCAR were synthesized and purified by the solid-phase peptide synthesis method.

The five segments of sequences are as follows:
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala,
A1-5-3: Gly-Pro-Pro-Gly-Ala-Thr-Gly-Phe-Pro, and
OSC^{pep}: Gly-Ala-Pro-Gly-Pro-Ala-Gly-Phe-Ala.

The third amino acid Pro in each sequence was hydroxylated, and the sixth amino acid Pro in A1-2-1 was hydroxylated.

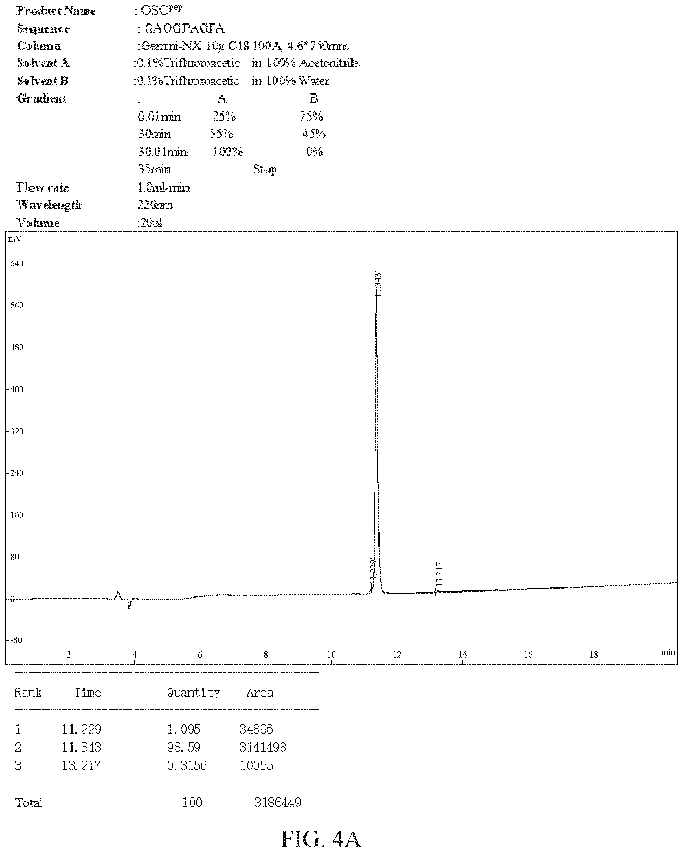

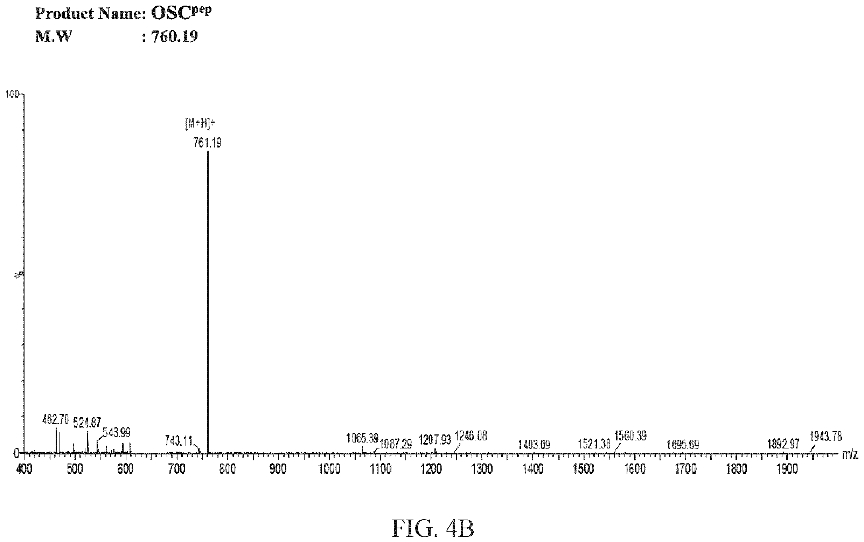

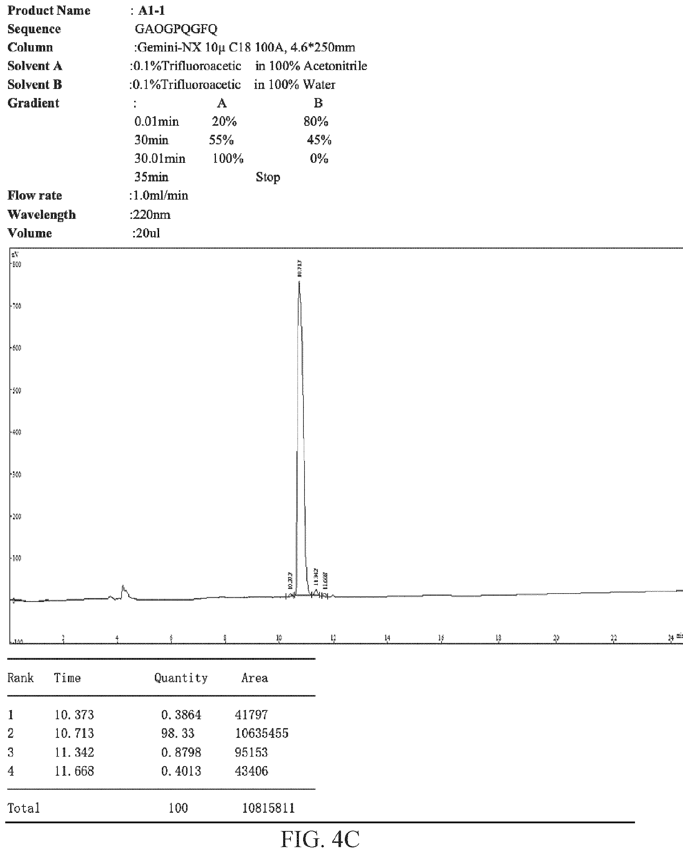

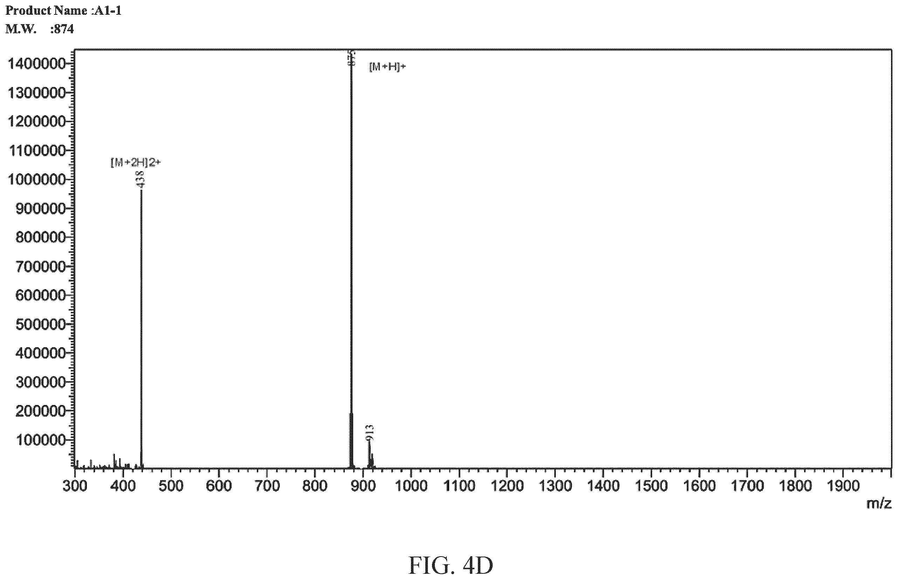

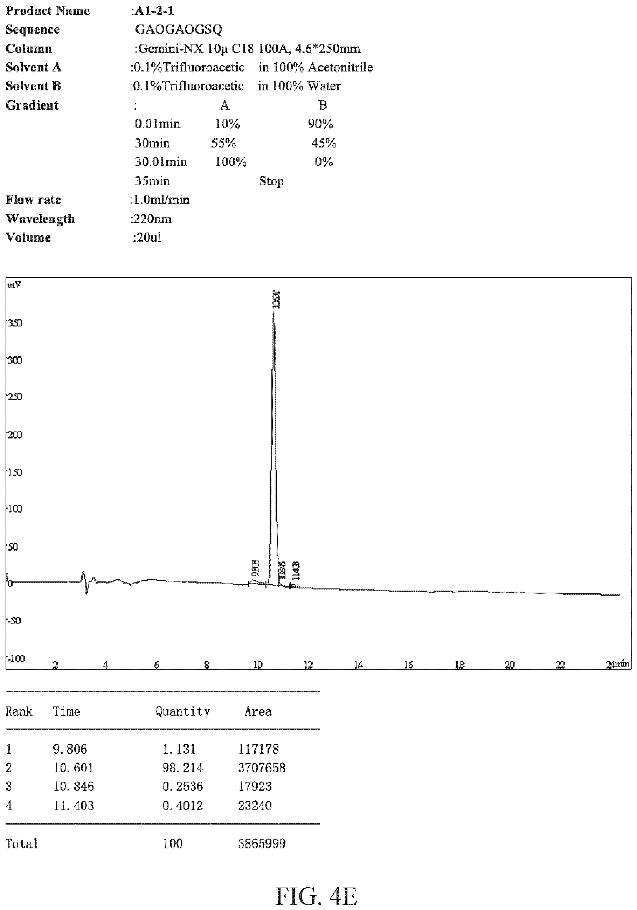

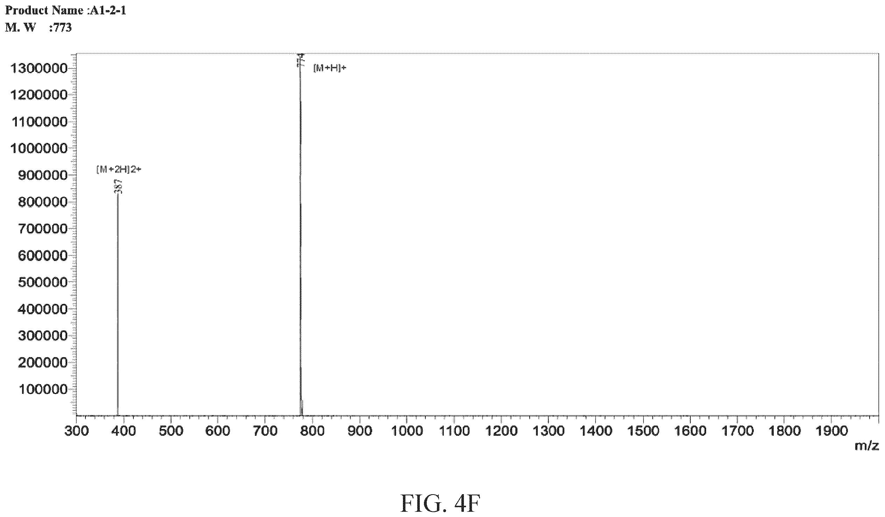

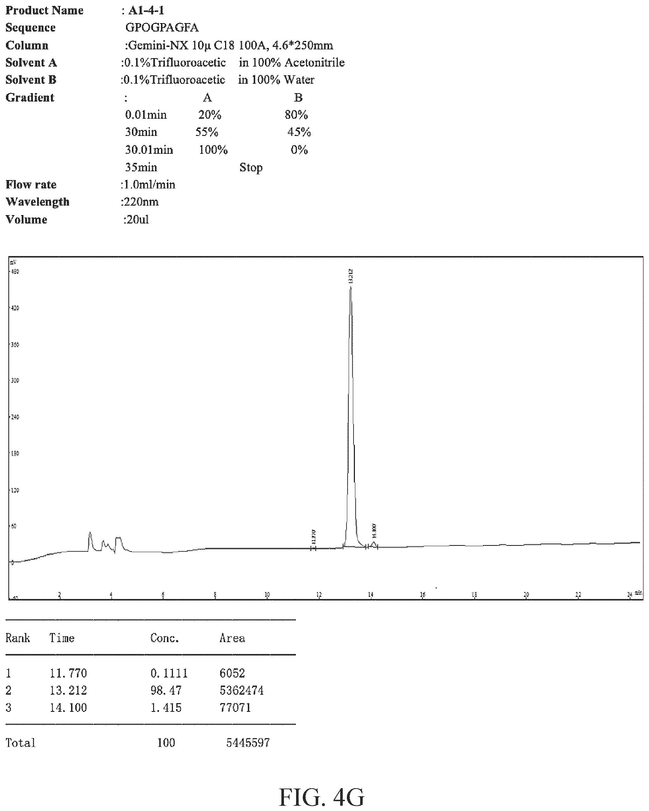

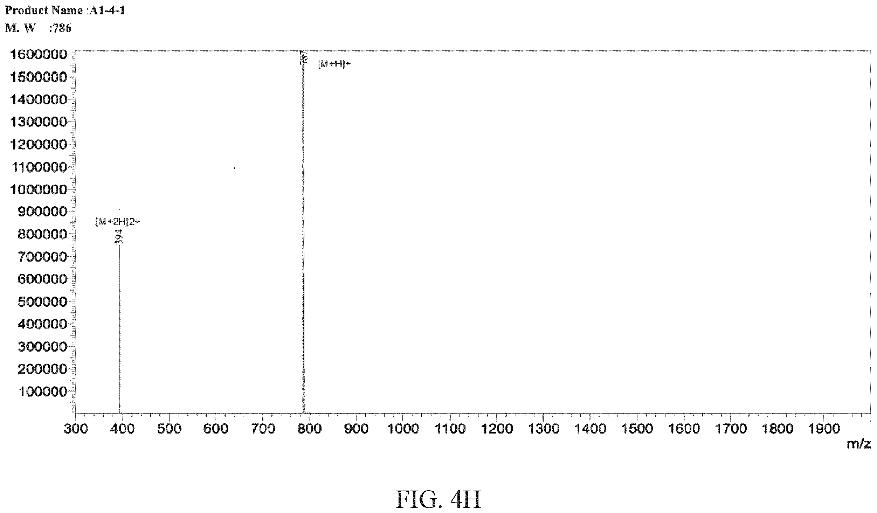

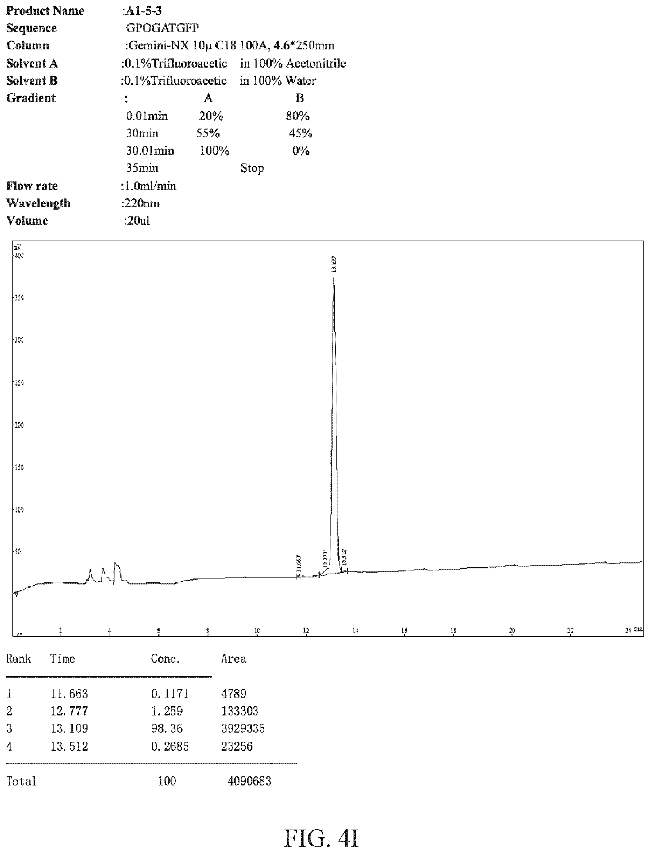

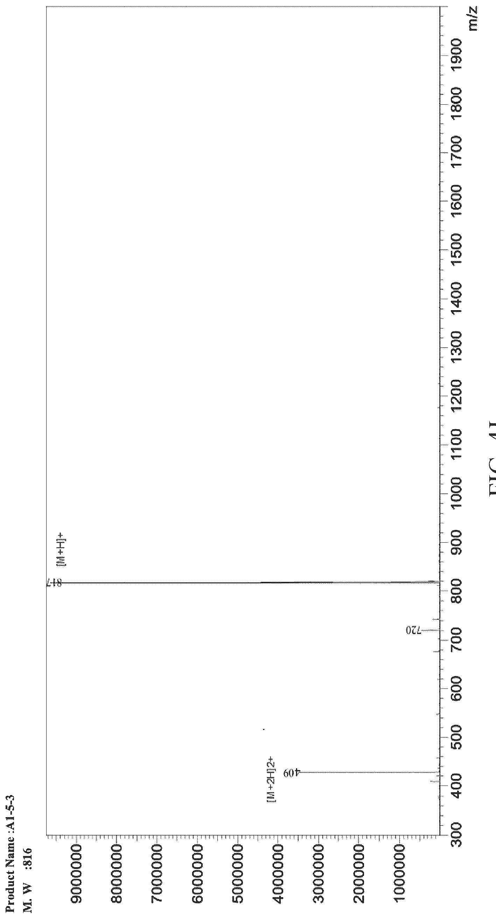

Fmoc-amino acid-OH and DIEA were added into Wang resin, and connection of the first amino acid was completed by mixing and shaking. After eluting Fmoc protective groups with 20% piperidine DMF, the second Fmoc-amino acid-OH, HBTU and DIEA were added and the second amino acid was connected by mixing and shaking. Then, the connection of the subsequent amino acids were completed in turn according to the method of the connecting the second amino acid. Finally, after eluting Fmoc protective groups, crude peptides were obtained by cutting peptides from the resin, and then purified by high performance liquid chromatography. After lyophilization, the purity was detected by LC-MS and was > 98% (FIG. 4).

### Embodiment 4

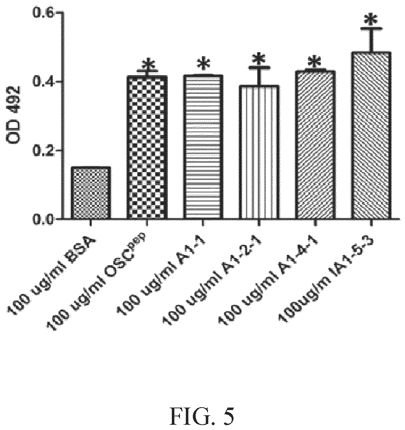

Solid-phase binding experiment shows that the type II collagen peptide (OSC^{pep}) and 4 segments of type I collagen peptides (A1-1, A1-2-1, A1-4-1, and A1-5-3) can bind to the collagen receptor OSCAR.
1) The extracellular domains of the mOSCAR was amplified by RT-PCR and inserted with expression vectors of His tag fusion proteins. Then, the constructed vectors were transfected into HEK293T cells for expression, and mOSCAR-His fusion protein were purified by ionexchange chromatography and molecular sieve chromatography.
2) Collagen peptides were dissolved with 10 mM acetic acid and wrapped to a 96-well microtitration plate overnight. After being sealed in phosphate buffer containing bovine serum albumin at room temperature for 1 hour, mOSCAR-His fusion protein was added and incubated at 37°C for 3 hours. Then, horseradish peroxidase-labeled goat anti-mouse IgG antibody that capable of reacting specifically with His added and incubated at room temperature for 1 hour. The binding mOSCAR-His fusion protein was detected by O-phenylenediamine dihydrochloride. The reaction was terminated by 3 M H2SO4, and the absorbance at 492 nm was measured. It was confirmed that the type II collagen peptide (OSC^{pep}) and 4 segments of type I collagen peptides (A1-1, A1-2-1, A1-4-1, A1-5-3) could bind to the mOSCAR (FIG. 5).

### Embodiment 5

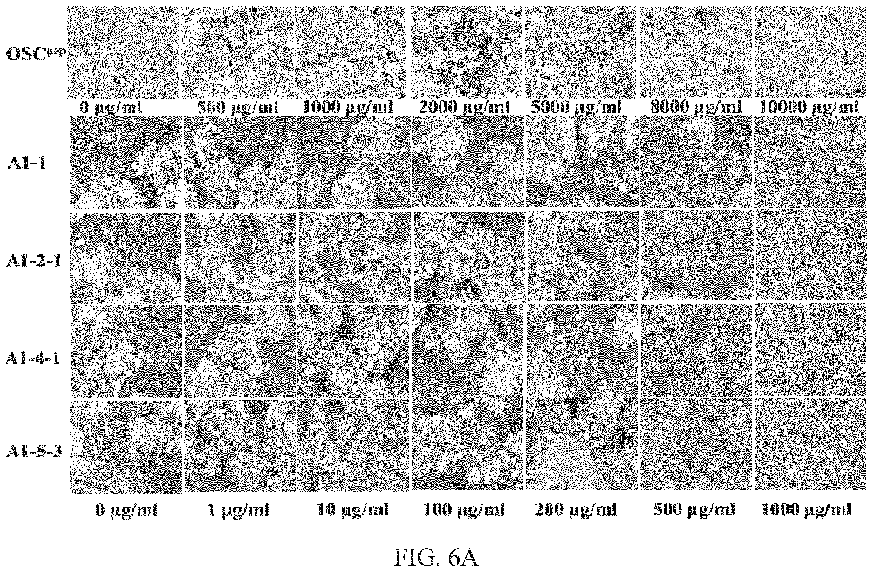

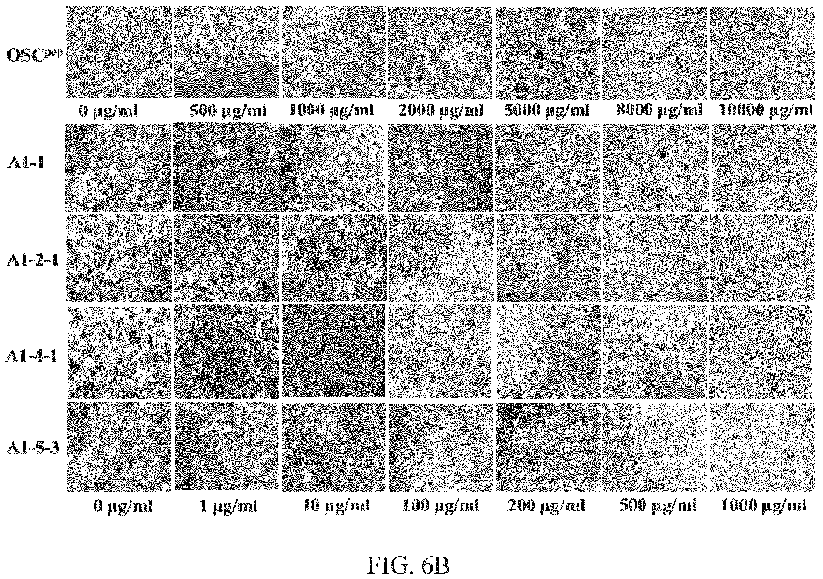

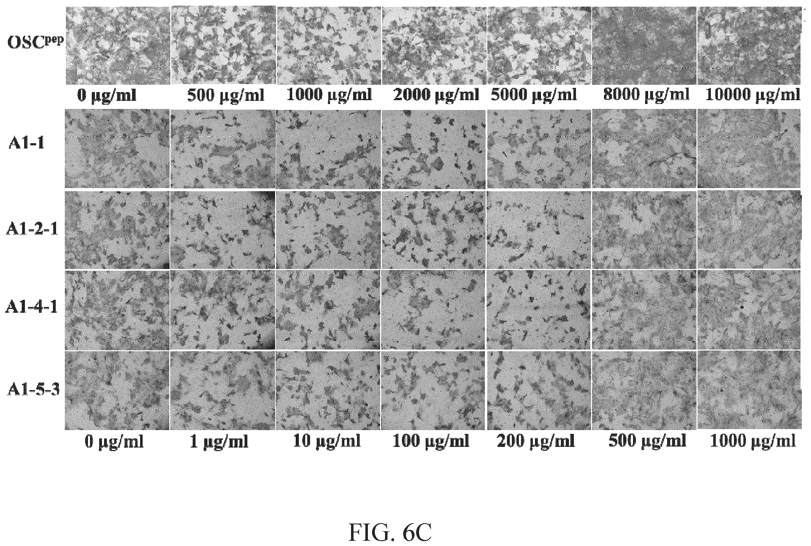

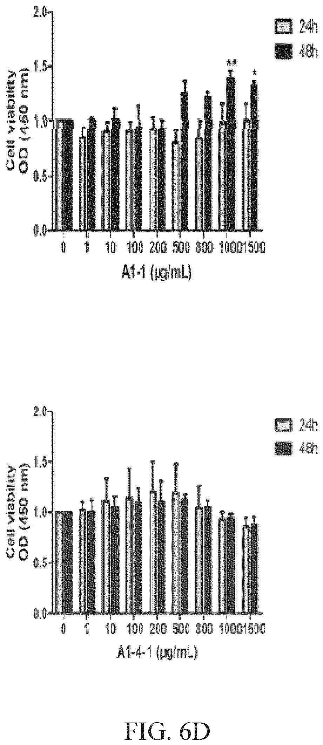

After verifying that the 5 segments of bone remodeling regulatory peptides have similar physical and chemical properties, and have the ability to bind to the mouse collagen receptor OSCAR, we carried out the experimental verification of this part. The *in vitro* cell experiment shows that the 5 segments of collagen peptides can concentration-specifically regulate differentiation and function of osteoclasts and osteoblasts without cytotoxicity.
1) Primary BMMs were cultured *in vitro* according to the method in Part 1 above. the A1-1/A1-4-1 collagen peptide was taken as an example for carrying out the cytotoxicity experiment. Primary BMMs were treated with different concentrations (0,1, 10, 100, 200, 500, 800, 1000, and 1500 µg/mL) of collagen peptide for 24 and 48 hours, and the cytotoxicity was determined by CCK-8 kit. It was confirmed that the above concentrations of the bone remodeling regulatory peptide had no toxic effect on cells (FIG. 6D).
2) Primary BMMs were treated with different concentrations of 5 collagen peptides, and induced to differentiate into osteoclasts by M-CSF and RANKL. The effect of different concentrations of collagen peptides on osteoclast differentiation was verified by TRAP staining. It was confirmed that all of the bone remodeling regulatory peptides could promote osteoclast differentiation at low concentration and inhibit osteoclast differentiation at high concentration (FIG. 6A).
3) Different concentrations of bone remodeling regulatory peptides-primary BMMs-bone slice culture system was constructed by using the above-mentioned method, and the cells in the system were induced to differentiate into osteoclasts by M-CSF and RANKL at the same time. The effect of collagen peptides on bone resorption of osteoclasts was verified by wheat germ agglutinin (WGA) staining. It was confirmed that all of the bone remodeling regulatory peptides could promote bone resorption of osteoclasts at low concentration and inhibit bone resorption of osteoclasts at high concentration (FIG. 6B).
   TRAP staining and the WAG straining show that the 4 segments of type I collagen peptides (A1-1, A1-2-1, A1-4-1, and A1-5-3) can promote osteoclast differentiation and bone resorption at low concentration (< 500 µg/mL, preferably 200 µg/mL), and can inhibit osteoclast differentiation and bone resorption at high concentration (≥ 500 µg/mL, preferably 500-1000 µg/mL); the type II collagen peptide (OSC^{pep}) can promote osteoclast differentiation and bone resorption at low concentration (< 8000 µg/mL, preferably 1000-5000 µg/mL) and can inhibit osteoclast differentiation and bone resorption at high concentration (≥ 8000 µg/mL, preferably 8000-10000 µg/mL).
4) Primary pre-osteoblasts were cultured *in vitro* according to the method in Part 1 above. Then, cells were treated with different concentrations of 5 segments of collagen peptides, and induced to differentiate into osteoblast by 10 mM β-sodium glycerophosphate, 50 µM vitamin C and 10⁻⁷ M dexamethasone at the same time. The effect of the collagen peptides on osteoblast differentiation was verified by ALP staining. It was confirmed that the bone remodeling regulatory peptides could inhibit osteoblast differentiation and bone formation at low concentration and promote osteoblast differentiation and bone formation at high concentration (FIG. 6C). ALP straining shows that the 4 segments of type I collagen peptides (A1-1, A1-2-1, A1-4-1, and A1-5-3) can inhibit osteoblast differentiation and bone formation at the concentration of < 500 µg/mL (preferably 200 µg/mL), and can promote osteoblast differentiation and bone formation at the concentration of ≥ 500 µg/mL (preferably 500-1000 µg/mL). The type II collagen peptide (OSC^{pep}) can inhibit osteoblast differentiation and bone formation at the concentration of < 8000 µg/mL (preferably 1000-5000 µg/mL), and can promote osteoblast differentiation and bone formation at the concentration of ≥ 8000 µg/mL (preferably 8000-10000 µg/mL).

### Embodiment 6

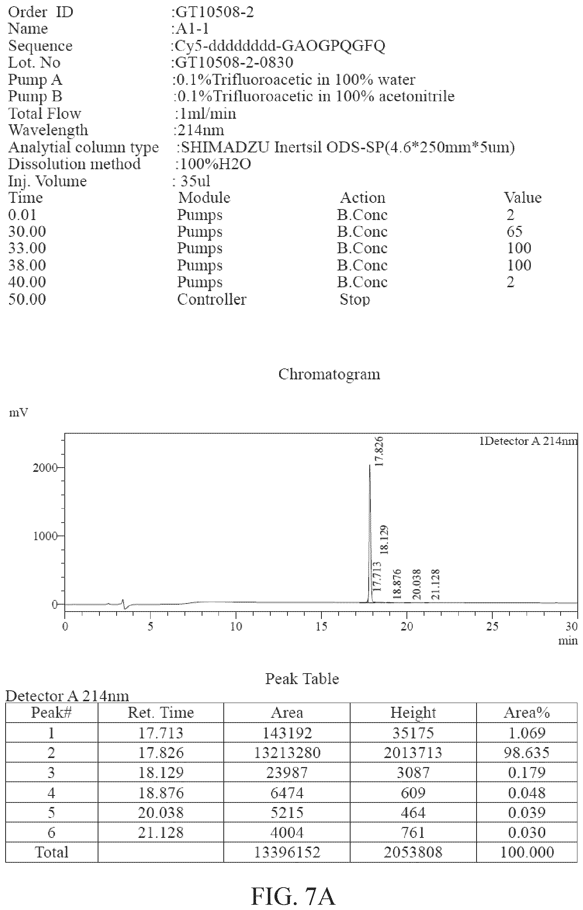

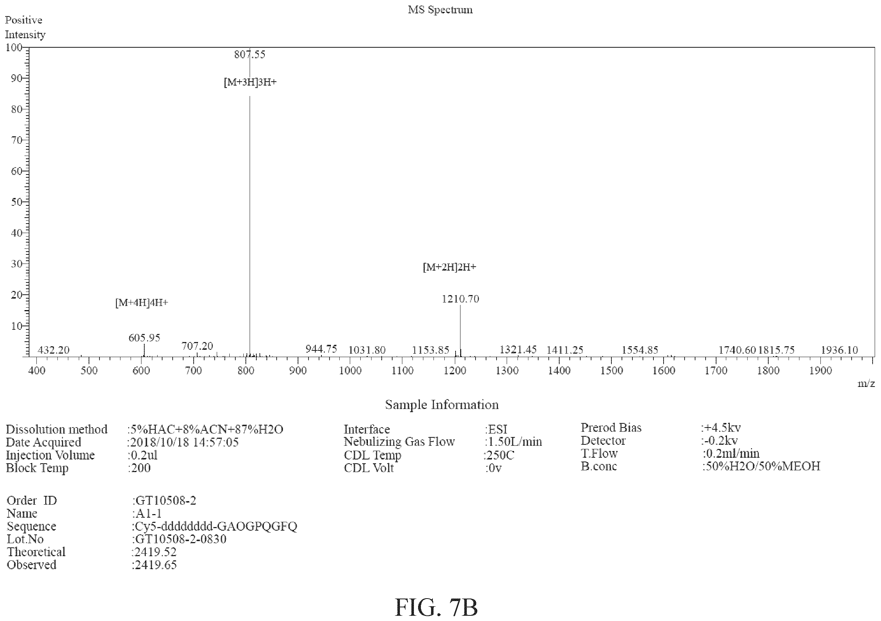

Cy5 fluorescence labeled-bone remodeling regulatory peptides that can target bone tissue were designed and synthesized, and its bone targeting property was determined by animal experiment *in vivo.*
1) The 5 segments of Cy5 fluorescence labeled bone remodeling regulatory peptides targeting the bone tissue were synthesized and purified by the solid-phase peptide synthesis method.
   Fmoc-amino acid-OH and DIEA were added into Wang resin, and connection of the first amino acid was completed by mixing and shaking. After eluting the Fmoc protective groups with 20% piperidine DMF, the second Fmoc-amino acid-OH, HBTU and DIEA were added and the second amino acid was connected by mixing and shaking. Then, the connection of the subsequent amino acids were completed in turn according to the method of the connecting the second amino acid. Then, after eluding the Fmoc protective groups, Cy5.0NHS fluorescence was added and react in anhydrous acetonitrile solution in the dark for 30 minutes. Finally, crude peptides were obtained by cutting peptides from the resin, and target peptides were purified by high performance liquid chromatography. After lyophilization, the purity was detected by LC-MS and was > 98%. The results of mass spectrometry and chromatography of the Cy5 labeled (Asp)8-type I collagen peptide (taking A1-1 as an example) are shown in FIG. 7
2) After verifying that the 5 segments of bone remodeling regulatory peptides have similar physical and chemical properties, and have the ability to bind to the mouse collagen receptor OSCAR, we took A1-4-1 as a representative to carry out experimental verification of this part. *In vivo* animal experiment confirmed that the bone targeting-bone remodeling regulatory peptides can specifically target bone tissue, and its half-life was determined.

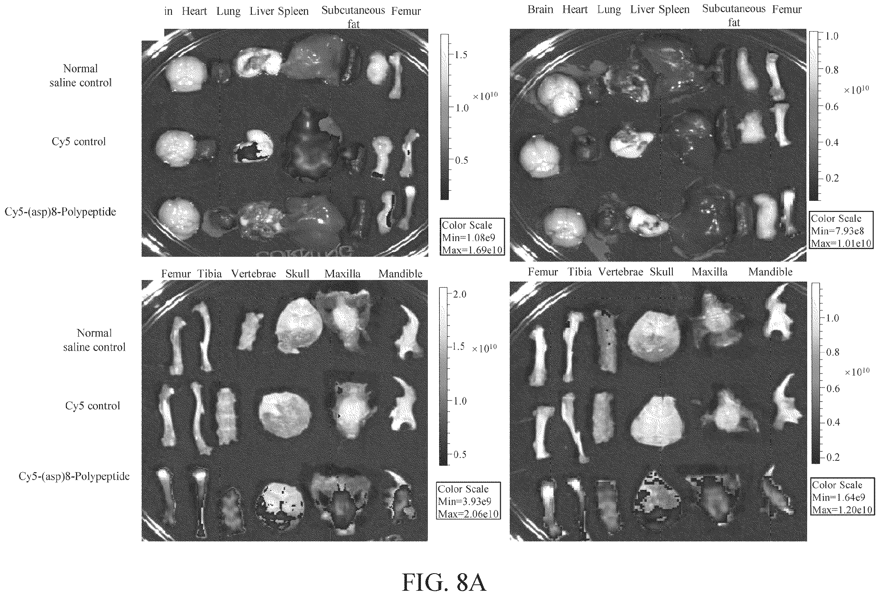

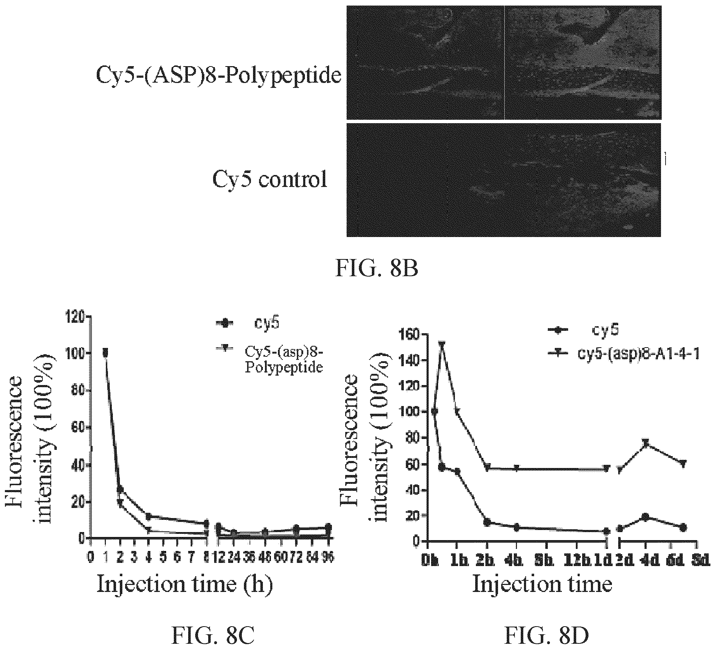

The Cy5 labeled-(Asp)8-bone remodeling regulatory peptides were injected into mice via tail vein. After 1 h, 2 h, 4 h, 8 h, 12 h, 1 d, 2 d, 4 d and 7 d of injection, tissues (brains, hearts, lungs, livers, spleens, subcutaneous fat, and bone tissues) were separated for fluorescence imaging. The result shows that compared with normal saline group and Cy5 control group, Cy5-(Asp)8-bone remodeling regulatory peptides group only express fluorescence in femurs, tibias, vertebrae, skulls, and alveolar bones at 1 h, 2 h, 4 h, 8 h, 12 h, 1 d, 2 d, 4 d, and 7 d of injection, but not in other tissues and organs (results of 2 h and 96 h are shown in FIG. 8). The femurs were taken for hard tissue section, and the expression of the Cy5 fluorescence in bone was observed by confocal microscope. The result shows that red fluorescence was expressed in the Cy5 labeled-(Asp)8-bone remodeling regulatory peptides group, but not in the Cy5 control group (FIG. 8B), indicating that Cy5 labeled-(Asp)8-bone remodeling regulatory peptides can specifically target bone tissues. Cy5 labeled-(Asp)8-bone remodeling regulatory peptides were injected into rats via tail vein. Blood samples were collected from tail vein at 1 h, 2 h, 4 h, 8 h, 12 h, 1 d, 2 d, 4 d and 7 d of injection, and serum was collected by centrifugation. The fluorescence in serum was quantified by fluorescence imager, which shows that the half-life of Cy5 labeled-(Asp)8-bone remodeling regulatory peptides in blood is 1.5 hours (FIG. 8C). The fluorescence of the bone tissues at each time point was quantified by fluorescence imager. The result shows that the half-life of Cy5 labeled-(Asp)8-bone remodeling regulatory peptides in bone is more than 7 days (FIG. 8D).

The bone remodeling regulatory peptides designed and synthesized in the present invention have a concentration-specific regulation effect on the differentiation and function of osteoclasts and osteoblasts. The bone remodeling regulatory peptides can specifically target the bone tissues by connecting (Asp)8, which is expected to be developed as a new clinical drug for the treatment of metabolic bone diseases such as osteoporosis.

## Claims

1. Bone remodeling regulatory peptides, wherein a core sequence is any one or more of the following:
Gly-Xaa-Pro-Gly-Xaa-Xaa-Gly-Xaa-Xaa,
wherein the second amino acid Xaa is Ala or Pro or Gly, the fifth amino acid Xaa is Pro or Ala, the sixth amino acid Xaa is Ala or Gln or Thr or Ser or hydroxylated Pro, the eighth amino acid Xaa is Phe or Ser or Asp or Tyr, the ninth amino acid Xaa is Ala or Gln or Pro or Arg or hydroxylated Pro, and the third amino acid Pro is hydroxylated.

2. The bone remodeling regulatory peptides according to claim 1, wherein the core sequence is any one or more of the following:
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala,
A1-5-3: Gly-Pro-Pro-Gly-Ala-Thr-Gly-Phe-Pro, and
OSC^{pep}: Gly-Ala-Pro-Gly-Pro-Ala-Gly-Phe-Ala.

3. The bone remodeling regulatory peptides according to claim 1, wherein the core sequence is any one or more of the following:
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala,
A1-5-3: Gly-Pro-Pro-Gly-Ala-Thr-Gly-Phe-Pro.

4. The bone remodeling regulatory peptides according to claim 1 or 2 or 3, comprising a series of peptides having a bone remodeling regulating function obtained by N-terminal or C-terminal modification, extension of an amino acid sequence, substitution and replacement of amino acids, and cyclization or chiral transformation on the basis of the core sequence.

5. The bone remodeling regulatory peptides according to claim 4, wherein the bone remodeling regulating function refers to controlling differentiation and function of osteoclasts and osteoblasts by regulating the concentration of bone remodeling regulatory peptides, thereby achieving the orderly regulation of bone resorption and bone formation.

6. The bone remodeling regulatory peptides according to claim 1 or 2 or 3 or 4, wherein an amino terminal of the amino acid sequence is connected with at least 2 aspartic acids, preferably 8 consecutive aspartic acids.

7. Application of any one of the bone remodeling regulatory peptides in claims 1-6 in preparing a preparation for promoting bone resorption and inhibiting bone formation, or a preparation for inhibiting bone resorption and promoting bone formation.

8. The application according to claim 7,
wherein when the bone remodeling regulatory peptides are any one or more of the following:
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala, and
A1-5-3: Gly-Pro-Pro-Gly-Ala-Thr-Gly-Phe-Pro, the concentration of the preparation for promoting bone resorption and inhibiting bone formation is < 500 µg/mL (preferably < 200 µg/mL); the concentration of the preparation for inhibiting bone resorption and promoting bone formation is ≥ 500 µg/mL (preferably 500-1000 µg/mL); and
when the bone remodeling regulatory peptide is OSC^{pep}: Gly-Ala-Pro-Gly-Pro-Ala-Gly-Phe-Ala, the concentration of the preparation for promoting bone resorption and inhibiting bone formation is < 8000 µg/mL (preferably 1000-5000 µg/mL); the concentration of the preparation for inhibiting bone resorption and promoting bone formation is ≥ 8000 µg/mL (preferably 8000-10000 µg/mL).

9. The application of the bone remodeling regulatory peptides in claims 1-6 in preparing a preparation for the treatment of metabolic bone diseases including osteoporosis and osteosclerosis.

10. The application according to claim 9,
wherein when the bone remodeling regulatory peptides are any one or more of the following:
A1-1: Gly-Ala-Pro-Gly-Pro-Gln-Gly-Phe-Gln,
A1-2-1: Gly-Ala-Pro-Gly-Ala-Pro-Gly-Ser-Gln,
A1-4-1: Gly-Pro-Pro-Gly-Pro-Ala-Gly-Phe-Ala, and
A1-5-3: Gly-Pro-Pro-Gly-Ala-Thr-Gly-Phe-Pro, the concentration of the preparation for osteoporosis treatment is ≥ 500 µg/mL (preferably 500-1000 µg/mL); the concentration of the preparation for osteosclerosis treatment is < 500 µg/mL (preferably < 200 µg/mL); and
when the bone remodeling regulatory peptide is OSC^{pep}: Gly-Ala-Pro-Gly-Pro-Ala-Gly-Phe-Ala, the concentration of the preparation for osteoporosis treatment is ≥ 8000 µg/mL (preferably 8000-10000 µg/mL); the concentration of the preparation for osteosclerosis treatment is < 8000 µg/mL (preferably 1000-5000 µg/mL).


SEQUENCE LISTING

<110>  THE SECOND XIANGYA HOSPITAL OF CENTRAL SOUTH UNIVERSITY

<120>  BONE REMODELING REGULATORY PEPTIDES AND APPLICATION THEREOF

<130>  GBCSRZ024

<150>  PCT/CN2019/100736
<151>  2019-08-15

<150>  CN 201910621375.2
<151>  2019-07-10

<160>  6

<170>  PatentIn version 3.5

<210>  1
<211>  9
<212>  PRT
<213>  Artificial Sequence

<220>
<223>  The sequence is synthesized


<220>
<221>  misc_feature
<222>  (2)..(2)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(9)
<223>  Xaa can be any naturally occurring amino acid

<400>  1

Gly Xaa Pro Gly Xaa Xaa Gly Xaa Xaa
1               5


<210>  2
<211>  9
<212>  PRT
<213>  Artificial Sequence

<220>
<223>  The sequence is synthesized


<220>
<221>  SITE
<222>  (3)..(3)
<223>  The third amino acid in the sequence (Pro) is hydroxylated

<400>  2

Gly Ala Pro Gly Pro Gln Gly Phe Gln
1               5


<210>  3
<211>  9
<212>  PRT
<213>  Artificial Sequence

<220>
<223>  The sequence is synthesized


<220>
<221>  SITE
<222>  (3)..(3)
<223>  The third amino acid in the sequence (Pro) is hydroxylated

<220>
<221>  SITE
<222>  (6)..(6)
<223>  The sixth amino acid in the sequence (Pro) is hydroxylated

<400>  3

Gly Ala Pro Gly Ala Pro Gly Ser Gln
1               5


<210>  4
<211>  9
<212>  PRT
<213>  Artificial Sequence

<220>
<223>  The sequence is synthesized


<220>
<221>  SITE
<222>  (3)..(3)
<223>  The third amino acid in the sequence (Pro) is hydroxylated

<400>  4

Gly Pro Pro Gly Pro Ala Gly Phe Ala
1               5


<210>  5
<211>  9
<212>  PRT
<213>  Artificial Sequence

<220>
<223>  The sequence is synthesized


<220>
<221>  SITE
<222>  (3)..(3)
<223>  The third amino acid in the sequence (Pro) is hydroxylated

<400>  5

Gly Pro Pro Gly Ala Thr Gly Phe Pro
1               5


<210>  6
<211>  9
<212>  PRT
<213>  Artificial Sequence

<220>
<223>  The sequence is synthesized


<220>
<221>  SITE
<222>  (3)..(3)
<223>  The third amino acid in the sequence (Pro) is hydroxylated

<400>  6

Gly Ala Pro Gly Pro Ala Gly Phe Ala
1               5